# EUROPEAN PATENT APPLICATION

(11) **EP 2 292 200 A1**
(43) Date of publication of application: **09.03.2011**
(21) Application number: 09754647.7
(22) Date of filing: 25.05.2009
(51) Int. Cl.: A61F 13/15, A61F 13/472, A61F 13/534

(54) **ABSORPTIVE ARTICLE AND SANITARY NAPKIN**

(30) Priority: 28.05.2008 JP 2008140053
(71) Applicant: Unicharm Corporation, Ehime 799-0111 (JP)
(72) Inventor: NODA, Yuki, Kanonji-shi Kagawa 769-1602 (JP); WADA, Mitsuhiro, Kanonji-shi Kagawa 769-1602 (JP); KOMATSU, Shinpei, Kanonji-shi Kagawa 769-1602 (JP)
(74) Representative: Knights, Rupert
(86) International application number: PCT/JP2009/059517
(87) International publication number: WO 2009/145139

(57) **Abstract**

An absorbent article (100) comprises a liquid-permeable front sheet (10), a liquid-impermeable back sheet (40), and an absorber (30) mounted between the top sheet (10) and the back sheet (40). The absorbent article (100) also comprises a front region (210), a central region (220), and a rear region (230) which are continuously arranged in the longitudinal direction of the absorber. An endothermic material (50) is mounted inside the absorber (30), in a mounting region at least including the central region (220) of the absorbent article.

## Description

### BACKGROUND OF THE INVENTION

### 1. Field of the Invention

The present invention relates to an absorbent article and a sanitary napkin each including a liquid permeable top sheet, a liquid impermeable back sheet, and an absorber interposed between the top sheet and the back sheet.

### 2. Description of the Related Art

Conventionally, in order to absorb menstrual blood excreted from a wearer, absorbent articles such as sanitary napkins have been widely used (for example, see Patent document 1). Generally, such an absorbent article has a liquid permeable top sheet, a liquid impermeable back sheet, and an absorber interposed between the top sheet and the back sheet.

### [Citation List]

### [Patent Literature]

Patent document 1:Japanese Patent Application Publication No.2001-190596
Patent document 2: Japanese Patent No.3922722

### SUMMARY OF THE INVENTION

Body fluid such as menstrual blood excreted from a wearer begins transpiring immediately after excretion to an absorbent article. For this reason, temperature and humidity in a space between a skin surface of the wearer and the absorbent article rise, thereby leading to a problem that the wearer feels stuffiness.

Thus, the present invention has been made in consideration of the above-mentioned problems. An object of the present invention is to provide an absorbent article and a sanitary napkin capable of suppressing stuffiness that the wearer feels when a body fluid such as menstrual blood or urine is excreted.

As disclosed in Japanese Patent No. 3922722, an absorbent article including an endothermic material whose temperature changes in response to an attachment of a body fluid (for example, endothermic reaction) has been known. However, such an absorbent article is a product for making the wearer recognize excretion of the body fluid. In other words, the absorbent article is used as a toilet training diaper for children, etc., and is not intended to suppress the stuffiness that the wearer feels.

Afirst aspect of the present invention is summarized by an absorbent article including a liquid permeable top sheet, a liquid impermeable back sheet, and an absorber interposed between the top sheet and the back sheet, wherein in the absorbent article, a front region, a central region, and a rear region are provided continuously in a longitudinal direction of the absorber, and an endothermic material is disposed inside the absorber and in an arrangement region including at least the central region of the absorbent article.

In the first aspect, a mixing percentage of the endothermic material in both end portions in a longitudinal direction of the absorbent article within the arrangement region may be lower than a mixing percentage of the endothermic material in a central portion in the longitudinal direction of the absorbent article within the arrangement region.

In the first aspect, the endothermic material is disposed in a plurality having different grain diameters may be disposed.

In the first aspect, the endothermic material having a grain diameter within a range of ± 100 µm of an overall average grain diameter is not more than 60% of the endothermic materials disposed inside the absorber.

In the first aspect, a grain diameter of the endothermic material disposed inside the absorber on a side facing the top sheet is smaller than a grain diameter of the endothermic material disposed inside the absorber on a side facing the back sheet.

A second aspect of the present invention is summarized by a sanitary napkin including a liquid permeable top sheet, a liquid impermeable back sheet, and an absorber interposed between the top sheet and the back sheet, wherein in the sanitary napkin, a front region, a central region, and a rear region are provided continuously in a longitudinal direction of the absorber, and an endothermic material is disposed inside the absorber in an arrangement region including at least the central region of the sanitary napkin.

In the second aspect, the sanitary napkin may further include a wing extending in a width direction, and a region in a longitudinal direction of the central region is a region in a longitudinal direction of the wing.

In the second aspect, in the sanitary napkin, an embossed groove may be formed in both side portions in a width direction of the absorber, the embossed groove is formed along a longitudinal direction of the sanitary napkin, and the endothermic material may be interposed between the embossed grooves.

As described above, according to the present invention, it is possible to provide an absorbent article and a sanitary napkin each of which can suppress the stuffiness that the wearer feels when a body fluid such as menstrual blood or urine is excreted.

### BRIEF DESCRIPTION OF THE DRAWINGS

[Fig. 1] Fig. 1 is a plan view of an absorbent article according to a first embodiment of the present invention.
[Fig. 2] Fig. 2 is a sectional view of the absorbent article according to the first embodiment of the present invention.
[Fig. 3] Fig. 3 is a graph showing grain size distribution of an endothermic material of the absorbent article according to the first embodiment of the present invention.
[Fig. 4] Fig. 4 is a sectional view of an absorbent article according to modification 1 of the present invention.
[Fig. 5] Fig. 5 is a plan view of an absorbent article according to a second embodiment of the present invention.
[Fig. 6A] Fig. 6A is a cross sectional view of a test device according to Example 1 of the present invention.
[Fig. 6B] Fig. 6B is a longitudinal sectional view of the test device according to Example 1 of the present invention.
[Fig. 7A] Fig. 7A is a graph that shows changes in humidity according to Example 1 of the present invention.
[Fig. 7B] Fig. 7B is a graph that shows changes in temperature according to Example 1 of the present invention.
[Fig. 8A] Fig. 8A is a graph that shows changes in humidity according to Example 1 of the present invention.
[Fig. 8B] Fig. 8B is a graph that shows changes in temperature according to Example 1 of the present invention.

### DETAILED DESCRIPTION OF THE PREFERRED EMBODIMENTS

### (Absorbent Article According to First Embodiment of the Present Invention)

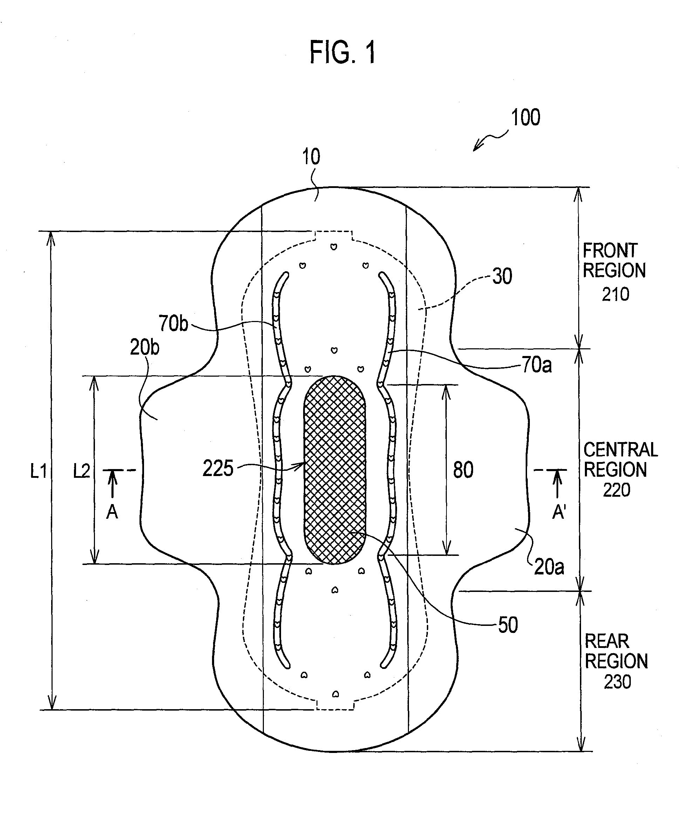

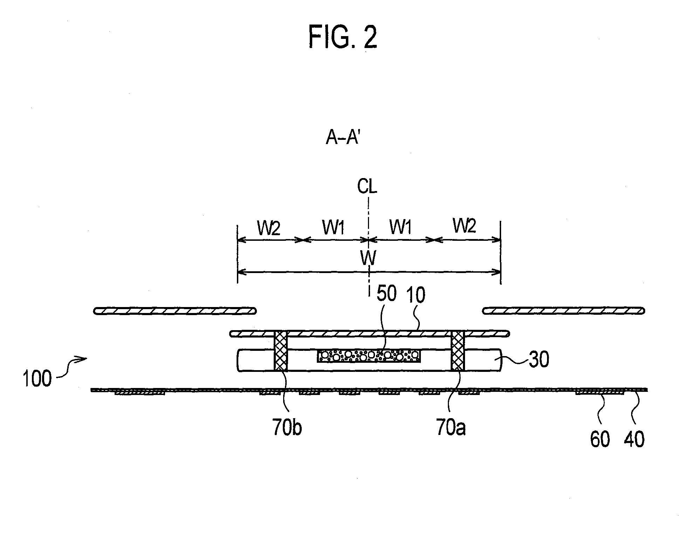

With reference to Figs. 1 to 2, a description will be given of an absorbent article according to a first embodiment of the present invention. Fig. 1 is a plan view of an absorbent article 100 according to the first embodiment of the present invention, and Fig. 2 is a sectional view of the absorbent article 100 taken along an A-A' line.

The absorbent article 100 is used as a sanitary napkin, for example. For storing in an individual packaging container and the like, the absorbent article 100 is folded inward in three or four, and is enclosed in the container. The whole shape of the absorbent article 100 may be rectangular, elliptical, gourd-shaped, etc., and is not particularly limited as long as the shape suits shapes of the wearer's body and underwear. Moreover, as an external dimension of the absorbent article 100, a dimension in a longitudinal direction is preferably in a range of "100 to 500 mm", and specifically, a range of "150 to 350 mm" is more preferable. Further, a dimension in a width direction is preferably in a range of "30 to 200 mm", and specifically, a range of "40 to 180 mm" is more preferable.

As shown in Fig. 1, in the present embodiment, the absorbent article 100 has a front region 210, a central region 220, and a rear region 230 continuously provided in a longitudinal direction (planar direction) of an absorber 30. The front region 210 is a region contacting a skin surface on a belly side of the wearer, the central region 220 is a region contacting a skin surface of a crotch part of the wearer, and the rear region 230 is a region contacting a skin surface on a buttocks side of the wearer.

Here, the central region 220 is a region to which a largest amount of a body fluid such as menstrual blood attaches when the wearer puts the absorbent article 100 on. For example, a region near the center in the width direction and in the longitudinal direction of the absorbent article 100 may be defined as the central region 220. When embossed grooves 70a to 70b (described later) are formed in the absorbent article 100, a crotch shaped portion 80 of the embossed grooves 70a to 70b (described later) may be defined as the central region 220, as shown in Fig. 1.

Moreover, in the absorbent article 100 according to the present embodiment, wings 20a to 20b extending in the width direction of the absorbent article 100 are formed so as to fix the absorbent article 100 to the underwear. When the wings 20a to 20b are thus formed, a region corresponding to the wings 20a to 20b may be defined as the central region 220. Specifically, a region in the longitudinal direction in which the wings 20a to 20b are provided may be defined as a region in a longitudinal direction of the central region 220. In the present embodiment, a description will be given based on an assumption that the region in the longitudinal direction in which the wings 20a to 20b are provided is defined as the region in the longitudinal direction of the central region 220.

As shown in Fig. 2, the absorbent article 100 has a liquid permeable top sheet 10, a liquid impermeable back sheet 40, and an absorber 30 interposed between the top sheet 10 and the back sheet 40. The top sheet 10 comes in contact with the skin surface of the wearer, and the back sheet 40 comes in contact with an underwear surface. In the absorbent article 100, it is preferable that the top sheet 10, the absorber 30, and the back sheet 40 be joined in order to prevent separation between layers. Specifically, in the absorbent article 100, it is preferable that the top sheet 10 and the back sheet 40 be joined to each other in a periphery portion of the absorber 30, so that the absorber 30 is enclosed therein. As a method for joining the top sheet 10 and the back sheet 40, it is possible to employ any processing of heat embossing, ultrasonic wave, or a hot melt adhesive or a combination of these.

As shown in Figs. 1 to 2, in the absorbent article 100, the embossed grooves 70a to 70b (the so-called hinge) are formed in the top sheet 10 and the absorber 30 in order to join the top sheet 10 and the absorber 30. The embossed grooves 70a to 70b are contoured in a crotch shaped pattern (embossed pattern) so that the absorbent article 100 can more fit the crotch part.

As shown in Figs. 1 to 2, the embossed grooves 70a to 70b are formed so as to extend in a longitudinal direction in both side portions in the width direction of the absorber 30. Here, in the present embodiment, when an interval in the width direction of the absorber 30 is W as shown in Fig. 2, each of the both side portions in the width direction of the absorber 30 is defined as a region shown as an interval W2 exceeding an interval W1 extending outward from the center of the absorber 30 in the width direction. For example, when the interval W1 is "10 mm", a region shown as the interval W2 exceeding "10 mm" extending outward from the center of the absorber 30 in the width direction is defined as each of the both side portions. Note that the both side portions are not limited to the content mentioned above. For example, a ratio of the interval W1 to the interval W2 is set to "1:1" or "2:1", and the both side portions each may be defined as a region of an interval W2 corresponding to this ratio. In addition, an interval in the width direction between the embossed groove 70a and the embossed groove 70b is preferably "20 to 60 mm".

Moreover, in the absorbent article 100, in order to prevent side leakage of the body fluid such as menstrual blood, gathers (not shown) including an elastic material such as a resilient material may be provided in the both side portions in the width direction of the absorber 30.

The top sheet 10 employs a nonwoven fabric as a base material. Note that the base material for the top sheet 10 is not particularly limited to a specific one, but any material can be used as long as the material is a sheet-like material having a liquid permeable structure such as a woven fabric or a perforated plastic sheet. Both natural fibers and chemical fibers can be used as a material for the woven fabrics or nonwoven fabrics. Examples of the natural fibers include cellulose such as crushed pulp and cotton. Examples of the chemical fibers include regenerated cellulose such as rayon and fibril rayon, semisynthetic cellulose such as acetate and triacetate, thermoplastic hydrophobic chemical fibers, and thermoplastic hydrophobic chemical fibers subjected to hydrophilization treatment. Examples of the thermoplastic hydrophobic chemical fibers include single fibers such as polyethylene (PE), polypropylene (PP), and polyethylene terephthalate (PET), fibers obtained by graft polymerization of polyethylene and polypropylene, and composite fibers of a sheath-core structure and the like.

In particular, as the method of web forming of nonwoven fabrics, any one of dry methods (the carding method, the spun bonding method, the melt-blowing method, the air-laid method, etc.) and wet methods or a combination of the above-mentioned methods may be used. Moreover, the method of bonding may be thermal bonding, needle punching, and chemical bonding, but is not particularly limited to these methods. Alternatively, spunlace formed in a sheet form by the hydroentangling method may be used as a nonwoven fabric.

As a perforated plastic sheet, perforated sheets made of a thermoplastic resin such as polyethylene, polypropylene, and polyethylene terephthalate, porous foam materials, or the like can be used. Moreover, a filler composed of titanium oxide, calcium carbonate, or the like may be added to the perforated plastic sheet in a range of 0.5 to 10%, if needed. As the perforated sheet, a perforated film obtained by making holes by perforation, heat embossing, or machining in a film made of a thermoplastic resin may be used. A composite sheet obtained by combining the film thus perforated with a nonwoven fabric may also be used.

The back sheet 40 has an adhesive part 60 adhering to the underwear of the wearer for preventing displacement disposed on a surface on the underwear side. A peeling sheet (not shown) is disposed by adhesion on an external surface of the adhesive part 60. A material which can be employed for the back sheet 40 includes, films mainly formed of polyethylene, polypropylene and the like, air permeable resin films, a combination of air permeable resin films joined to a nonwoven fabric such as spun bond, and spunlace and the like, plural layers of SMS, and others. Taking account flexibility of a level which does not reduce the fittingness, it is preferable that the back sheet 40 employ a film mainly formed of a low density polyethylene (LDPE) resin and having a basis weight within "15 to 30 g/m²", for example.

The absorber 30 may be a product formed of a hydrophilic fiber or a polymer covered with a coating material, or may be an air-laid sheet formed into a sheet form by an air laid method.

Examples of the hydrophilic fibers include cellulose such as crushed pulp and cotton, regenerated cellulose such as rayon and fibril rayon, semisynthetic cellulose such as acetate and triacetate, granular polymers, fibrous polymers, thermoplastic hydrophobic chemical fibers, and thermoplastic hydrophobic chemical fibers subjected to hydrophilization treatment. These can be used alone or being combined. Out of these, in consideration of lower costs and workability of forming the absorber, it is preferable that crushed pulp be used.

As an example of the polymers, granular polymers such as sodium acrylate copolymer having absorbency and hygroscopicity are generally used. Alternatively, in order to obtain other properties, a granular deodorant material such as silver, copper, zinc, silica, activated carbon, aluminosilicate compounds, and zeolite may be used.

For the coating material, for example, a woven fabric, a nonwoven fabric, or the like of any type without particular limitation is usable as long as the fabric is liquid permeable and has barrier properties sufficient to prohibit a high polymer absorbent and an endothermic material (described later) from passing through the fabric. Both natural fibers and chemical fibers are usable as a material for the woven fabrics and the nonwoven fabrics. Examples of the natural fibers include cellulose such as crushed pulp and cotton. Examples of the chemical fibers include regenerated cellulose such as rayon and fibril rayon, semisynthetic cellulose such as acetate and triacetate, thermoplastic hydrophobic chemical fibers, and thermoplastic hydrophobic chemical fibers subjected to hydrophilization treatment.

For web forming of a nonwoven fabric, either of dry methods (the carding method, the spun-bonding method, the melt-blowing method, the air-laid method, etc.) and wet methods may be performed, or the above-mentioned methods may be performed in combination. Moreover, the method of bonding may be thermal bonding, needle punching, and chemical bonding, but is not particularly limited to these methods. Alternatively, spunlace formed in a sheet form by the hydroentangling method may be used. Out of these, in consideration of lower cost and higher barrier properties, it is preferable to use a tissue mainly composed of crushed pulp and formed by the wet method.

When an air-laid sheet is used as the absorber 30, it is preferable that the thickness be 0.3 to 5.0 mm. Examples of the air-laid sheet include an air-laid sheet formed of fibers and a granular polymer in a sheet form by use of a binder and the like. In the air-laid sheet, the granular polymer may be distributed as a layer, or may be concentrated in a thickness direction.

Moreover, embossing may be formed in the absorber 30 in order to prevent deformation and twist during wearing, or in order to adjust a thickness. The absorber 30 can be embossed by passing between a patterned embossing roll and a flat roll. While either a lattice pattern, a dot pattern, and a wave pattern is employed as a pattern of the embossing roll, a lattice pattern is preferable because of its thickness adjustability.

A dimension in the longitudinal direction of the absorber 30 is preferably in a range of "90 to 490 mm", and specifically, it is more preferable in a range of "140 to 340 mm". Moreover, a dimension in the width direction is preferably in a range of "25 to 100 mm", and specifically, it is more preferable in a range of "35 to 80 mm".

As shown in Fig. 2, an endothermic material 50 is disposed in the absorber 30. When coming into contact with fluids such as body fluids, the endothermic material 50 is water-soluble and causes endothermic reaction to absorb surrounding heat energy. Examples of the endothermic material 50 include water-soluble materials that cause the endothermic reaction by hydration, such as potassium chloride, sodium chloride, sodium acetate, potassium nitrate, urea, sodium bicarbonate, xylitol, and trehalose. Out of these, from a viewpoint of irritativeness to the skin at the time of dissolution and stability such as long term storage, potassium chloride is preferable.

### (Arrangement Area of Endothermic Material)

A description will be given of an arrangement region of the endothermic material 50 in the absorbent article 100 according to the present embodiment. For an efficient generation of the endothermic reaction with excreted menstrual blood, it is preferable that an arrangement region 225 of the endothermic material 50 include at least a region contacting the crotch part of the wearer.

In the present embodiment, the endothermic material 50 is disposed in an arrangement region including at least a central region 220 in the longitudinal direction (planar direction) of the absorbent article 100, and is disposed inside the absorber 30 in the thickness direction of the absorbent article 100. A region in a longitudinal direction of the central region 220 corresponds to a region in the longitudinal direction of the wings 20a to 20b. Moreover, the endothermic material 50 is disposed in a region between the embossed groove 70a and the embossed groove 70b in the width direction (planar direction) of the absorbent article 100.

In the example of Fig. 1, the arrangement region 225 in the planar direction of the endothermic material 50 is indicated by a mesh pattern. Here, as shown in Fig. 1, the arrangement region including at least the central region 220 may be a region within the central region 220, or may be a region including a part of the central region 220 and an region outside of the central region 220. As shown in Fig. 1, when a length in the longitudinal direction of the absorber 30 is defined as "L1", it is preferable that a length in the longitudinal direction of the arrangement region 225 be a length "L2" having a 30 to 50% length of "L1".

Here, assume if the length in the longitudinal direction of the arrangement region 225 is less than 30% of the length in longitudinal direction of the absorbent article 100. In that case, when an amount of menstrual blood is less, or when the wearer does not wear the absorbent article 100 on an appropriate position, the endothermic material 50 may have difficulty in causing the endothermic reaction.

On the other hand, when the length in the longitudinal direction of the arrangement region 225 is not less than 50%, the endothermic material 50 may cause the endothermic reaction with sweat or the like excreted from the crotch part of the wearer other than menstrual blood. Accordingly, chilliness may be given to the wearer more than needed.

Therefore, it is preferable that the length in the longitudinal direction of the arrangement region 225 be 30 to 50% of the length in the longitudinal direction of the absorbent article 100.

More preferably, the arrangement region 225 of the endothermic material 50 includes not only the central region 220 but also the center in the longitudinal direction of the absorbent article 100. The center of the absorbent article 100 may be the center of the entire absorbent article 100, or may be a point on the center line that divides the wings 20a and 20b equally in the longitudinal direction. When the both side portions of the absorber 30 are formed to be curved in a form projected inward in the width direction, the center in the longitudinal direction of the absorbent article 100 may be a narrowest portion in the width of the absorber 30.

Moreover, when a middle higher part projecting in the thickness direction is formed in the absorbent article 100, the center of the absorbent article 100 may be the center in a planar direction of the middle higher part. The middle higher part may be formed by laminating multiple absorbers 30 having different sizes, or may be formed so as to have its basis weight of the absorber 30 larger than basis weights in other regions.

Moreover, a density of the endothermic material 50 of the arrangement region 225 may be uniform on the entire surface of the absorber 30, or may be formed so as to be high in the central region 220.

It is preferable that as for a position in the thickness direction, the endothermic material 50 is disposed in a position where uncomfortable feelings to the wearer such as chilliness by the endothermic reaction of the endothermic material 50 can be avoided. The endothermic material 50 may be disposed, for example, between the top sheet 10 and the absorber 30 or inside the top sheet. However, it is preferable that the endothermic material 50 be placed inside the absorber 30 in order not to repeat the endothermic reactions in a long period of time or not to give chilliness.

Moreover, the endothermic material 50 may be disposed at a uniform density in the thickness direction of the absorber 30. In order to reduce temperature even with a little amount of the excreted body fluid such as menstrual blood, it is preferable that the endothermic material 50 be disposed in a position on the skin surface side in the thickness direction of the absorber 30.

### (Grain Diameter of Endothermic Material)

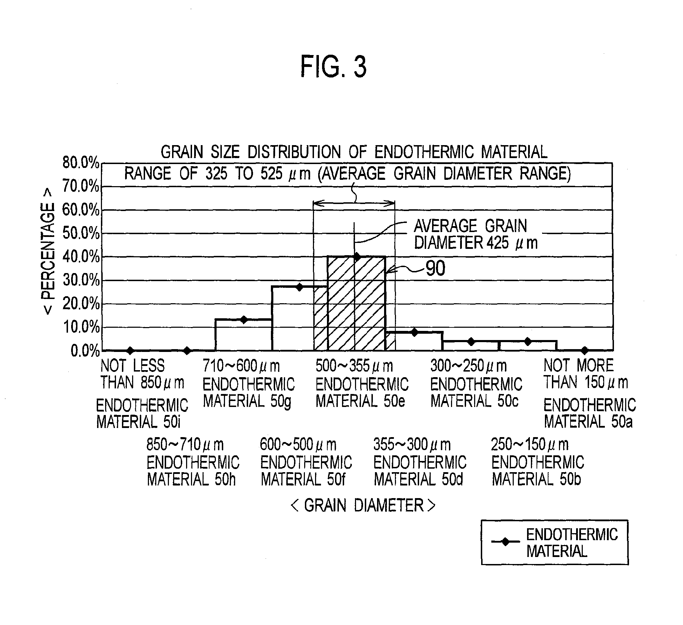

The grain diameter of the endothermic material 50 according to the present embodiment will be described with reference to Fig. 3. Fig. 3 is a graph that shows grain size distribution of the endothermic material according to the present embodiment. In Fig. 3, an abscissa shows a grain diameter and an ordinate shows a ratio (%) to an overall weight.

Several kinds of endothermic materials each having a different grain diameter are disposed in the endothermic material 50 according to the present embodiment. Specifically, the endothermic material 50 includes endothermic materials 50a to 50i having different grain diameters from each other, as shown in Fig. 3.

Here, in the present embodiment, the grain diameter of the endothermic material 50a is less than "150 µm", the grain diameter of the endothermic material 50b is "150 µm" or more but less than "250 µm", the grain diameter of the endothermic material 50c is "250 µm" or more but less than "300 µm", the grain diameter of the endothermic material 50d is "300 µm" or more but less than "355 µm", the grain diameter of the endothermic material 50e is "355 µm" or more but less than "500 µm", the grain diameter of the endothermic material 50f is "500 µm" or more but less than "600 µm", the grain diameter of the endothermic material 50g is "600 µm" or more but less than "710 µm", the grain diameter of the endothermic material 50h is "710 µm" or more but less than "850 µm", and the grain diameter of the endothermic material 50i is "850 µm" or more. Moreover, in the example of Fig. 3, an overall average grain diameter is "425 µm".

Moreover, as for the endothermic materials 50 in the present embodiment, the endothermic materials having a grain diameter within the range of ± 100 µm of the overall average grain diameter (within the range of the average grain diameter) are 60% or less (60% or less of a weight of the overall weight) of all (overall weight) of the endothermic materials disposed inside the absorber 30.

Specifically, in the example of Fig. 3, the range of ± 100 µm of the average grain diameter "425 µm" is the range of "325 µm" to "525 µm". In the present embodiment, a description will be given by defining this range as the range of the average grain diameter. Moreover, in the example of Fig. 3, the endothermic material 50d, a part of the endothermic material 50c, and a part of the endothermic material 50e correspond to the endothermic material within the range of the average grain diameter, and are shown as a region 90 by a slash pattern in Fig. 3.

Here, in Fig. 3, the whole weight of the endothermic material 50 and the whole area of the graph have a one-to-one correspondence. Therefore, in Fig. 3, a ratio of an area of the region 90 to the whole area of the graph shows a ratio of a weight of the endothermic material within the range of the average grain diameter to the whole weight of the endothermic material 50. In other words, in the present embodiment, the grain diameter of the endothermic material is selected so that the area of the region 90 shown in Fig. 3 may be not more than 60% of the whole area of the graph.

In this way, in the endothermic material 50 according to the present embodiment, the ratio of the endothermic material within the range of the average grain diameter is set to be not more than 60%, and at least not less than 40% of all is distributed in the endothermic materials of other grain diameters.

Here, when components of the endothermic materials are the same, the endothermic material having a larger grain diameter takes more time until completely dissolves. This is because the endothermic material begins to dissolve from an uppermost surface of the grains when dissolving by hydration, and therefore, the grain having larger diameter, that is, having larger volume takes more time to completely dissolve.

In order to cause the endothermic reaction even in the case of long-time excretion or repeated excretion, a larger grain diameter is more preferable. However, if the absorber 30 includes only an endothermic material having a larger grain diameter, rigidity of the absorber 30 increases, and therefore, feeling of foreign objects is given to the wearer.

For example, when the ratio of the endothermic material within the range of the average grain diameter is larger than 60% of all the endothermic material 50, a larger amount of the endothermic material having a larger grain diameter is included, so that feeling of foreign matters is given to the wearer. When a larger amount of the endothermic material having a smaller grain diameter is included, it becomes difficult to cause the endothermic reaction for a long time. Therefore, the ratio of the endothermic material within the range of the average grain diameter mentioned above is preferably a ratio of not more than 60% of all the endothermic material 50, and specifically, a ratio of not more than 50%.

In other words, in order to cause the endothermic reaction in the endothermic material 50 for a long time and to avoid excessive increase in rigidity of the absorber 30, it is preferable that the endothermic materials 50a to 50i having different grain diameters be mixed. Although the average grain diameter will not be particularly limited, the range of 300 to 800 µm, and specifically, the range of 350 to 600 µm is preferable from a viewpoint of feeling of foreign objects and a viewpoint of productivity. The grain diameter of the endothermic material will not be limited to the range in the example of Fig. 3.

### (Configuration of Endothermic Material in Absorber)

A description will be given of a configuration of the endothermic material 50 according to the present embodiment. As for the endothermic material 50, a sheet covering only the endothermic material 50 with a coating material may be disposed on an upper surface of the absorber 30, or the endothermic material 50 may be disposed as a layer between pulps. Further, the endothermic material 50 may be sandwiched between the top sheet 10 and the absorber 30.

Here, since the endothermic material 50 is water-soluble, for example, when a region of the endothermic material 50 where a diameter of a grain is small dissolves at a first excretion of menstrual blood, a space is generated in the endothermic material 50. In this case, when the menstrual blood is repeatedly excreted, it becomes difficult for the menstrual blood to move from the top sheet 10 to the back sheet 40. As a result, the menstrual blood stagnates in the top sheet 10, and thus the wearer may feels uncomfortable.

As mentioned above, it is preferable that the endothermic material 50 be blended with pulp and the like, and be dispersed to some extent. Specifically, the absorber 30 is preferably obtained by mixing pulp in a rage of 60 to 98%, the granular endothermic material 50 in a range of 40 to 2%, and a granular polymer in a range of 20 to 0%, the mixture is then covered with a tissue, and subsequently the mixture is formed into a sheet having a basis weight of 100 to 2,000 g/m² and a height of 1 to 50 mm by embossing. Embossing is performed for preventing deformation of the absorber, and an embossed area rate is preferably in a range of 10 to 100%, and specifically, in a range of 30 to 80%.

It is preferable that the endothermic material 50 in the absorber 30 has a configuration which allows the endothermic material 50 to repeat the endothermic reactions by the body fluid such as menstrual blood in a long period of time, or a configuration that gives no chilliness. Specifically, for an upper layer material (top sheet 10 side), pulp of 50 to 150 gsm and potassium chloride of 0.5 to 3.0 g are blended. For a lower layer material (back sheet 40 side), pulp of 50 to 200 gsm and a high polymer absorbent of 0.1 to 0.5 g are blended. Then, the upper layer material and the lower layer material are layered, covered with a tissue, and subsequently, embossed. This may be used as the endothermic material 50.

### (Mixing Amount of Endothermic Material)

A description will be given of a mixing amount of the endothermic material 50 in the absorber 30 according to the present embodiment. The mixing amount of the endothermic material 50 is influenced by the variety of heat of dissolution and solubility, which depend on a kind of the endothermic material 50, and also by an arrangement position of the endothermic material 50. Here, in consideration of suppressing sharp increases in temperature and humidity in the space between the skin surface and the absorbent article 100 and of giving no uncomfortable feelings to the wearer such as chilliness, a weight of the endothermic material 50 per sheet in the absorbent article 100 is preferably in a range of 0.1 to 10 g, and more specifically, in a range of 0.5 to 5 g.

As an example, a description will be given of a case where a sanitary napkin is used as the absorbent article 100 and potassium chloride is used as the endothermic material 50.

It is known that an average absorbed amount per a napkin in a day (the first to third day) during a menstrual period when much menstrual blood is excreted is generally approximately 6.0 ml. It is also known that solubility of potassium chloride is approximately 27.0% in an experiment liquid (physiological saline) adjusted to 37°C ± 5°C.

From these, potassium chloride that can dissolve to 6.0 ml of menstrual blood is approximately 2.0 g, and even when not less than potassium chloride 2.0g is mixed, the excessive potassium chloride does not cause the endothermic reaction. Accordingly, when potassium chloride is used in sanitary napkins for days with much menstrual blood loss, a mixing amount of potassium chloride is preferably in a range of 0.5 to 3.0 g, and more preferably in a range 1.0 to 2.0 g.

According to the absorbent article 100 of the first embodiment of the present invention, the endothermic material 50 is disposed inside the absorber 30. Accordingly, when the body fluid such as menstrual blood is excreted, the absorbent article 100 prevents the increases in temperature and humidity of the absorber 30 by the endothermic reaction of the endothermic material 50. In other words, the absorbent article 100 can suppress the stuffiness that the wearer feels when the body fluid such as menstrual blood and urine is excreted.

Further, when the water-soluble material used as the endothermic material 50 is dissolved into the blood, the solute concentration in the blood plasma increases. Therefore, the osmotic pressure of the blood plasma increases, and the water included in the red blood cell is discharged. This results in contraction of the red blood cell. Thus, the volume of the red blood cell decreases. When the volume of the red blood cell is decreased in this manner, surface tension of the blood increases, thereby contact angle of the blood with respect to the top sheet 10 increases. In other words, leakage probability of the red blood cell decreases.

As described above, when the water-soluble material is dissolved into the blood that has once passed through the top sheet 10, the leakage probability of the blood decreases. Therefore, the blood is not easily returned to the skin surface through the top sheet 10. Thus, the wearer can feel sense of dryness.

According to the absorbent article 100 of the first embodiment of the present invention, the endothermic material 50 does not come in contact with the skin surface of the wearer. Therefore, it is possible to suppress uncomfortable feelings such as chilliness given to the wearer due to the endothermic reaction of the endothermic material 50. Further, it is possible to suppress uncomfortable feelings given to the wearer due to stagnation of the body fluid in the top sheet 10 such as menstrual blood, of which the temperature has been reduced by the endothermic material 50.

Moreover, according to absorbent article 100 of the first embodiment of the present invention, the endothermic material 50 is disposed in the region including at least the central region 220 of the absorbent article 100. Additionally, in the present embodiment, the central region 220 corresponds to the region of the wings 20a to 20b in the longitudinal direction.

Here, the absorbent article 100 is generally used so that a region between the wing 20a and wing 20b may come in contact with the crotch part when the absorbent article 100 is put on. Accordingly, by defining the region of the wings 20a to 20b in the longitudinal direction as the central region 220 and disposing the endothermic material 50 in the region including at least the central region 220, the body fluid from the wearer such as menstrual blood can be made to attach to the endothermic material 50 more securely so as to cause the endothermic reaction.

Moreover, in the absorbent article 100 according to the first embodiment of the present invention, the embossed grooves 70a to 70b are formed in order to join the top sheet 10 and the absorber 30. The endothermic material 50 is disposed in the region between the embossed groove 70a and the embossed groove 70b.

Accordingly, in the absorbent article 100 according to the first embodiment of the present invention, the top sheet 10 and the absorber 30 are formed so as not to easily separate from each other. For this reason, the body fluid from the wearer such as menstrual blood can be made to attach to the endothermic material 50 more securely to cause the endothermic reaction. Further, according to the absorbent article 100, the endothermic material 50 is disposed in the region between the embossed groove 70a and the embossed groove 70b. Accordingly, even when the absorbent article 100 is twisted, endothermic material 50 is less likely to come out of the absorber 30.

According to the absorbent article 100 of the first embodiment of the present invention, the endothermic material 50 is formed so that the ratio of the endothermic material within the range of ± 100 µm of the average grain diameter (weight ratio to the overall weight) may be not more than 60%, and at least not less than 40% of all the endothermic material having other grain diameters may be distributed. Therefore, according to the absorbent article 100, the endothermic material 50 can cause the endothermic reaction for a long time, and can suppress excessive increase in rigidity of the absorber 30.

### (Modification 1)

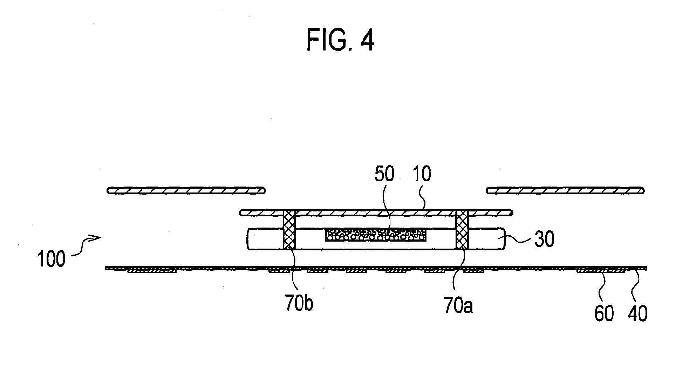

With reference to Fig. 4, a description will be given of an absorbent article according to modification 1 of the present invention focusing on differences from the absorbent article according to the first embodiment of the present invention.

As shown in Fig. 4, in the absorbent article 100 according to the modification 1, the grain diameter of the endothermic material 50 disposed on the top sheet 10 side of the absorber 30 is smaller than the grain diameter of the endothermic material 50 disposed on the back sheet 40 side of the absorber 30.

Specifically, in the absorbent article 100 according to the modification 1, the endothermic material 50a to 50d shown in Fig. 3 is disposed on the top sheet 10 side of the absorber 30, and the endothermic material 50e to 50i shown in Fig. 3 is disposed on the back sheet 40 side of the absorber 30.

According to the absorbent article 100 of the modification 1, the endothermic material having a smaller grain diameter is disposed on the top sheet 10 side. Accordingly, the absorbent article 100 is less likely to give the feeling of foreign objects due to the endothermic material 50 to the wearer.

### (Modification 2)

An absorbent article according to modification 2 of the present invention will be described focusing on differences from the absorbent article according to the first embodiment of the present invention.

Several kinds of the endothermic materials having a different solubility are disposed in the endothermic material 50 according to the modification 2. Specifically, as the several kinds of the endothermic materials having different solubility, the endothermic material 50 includes an endothermic material made of potassium chloride, an endothermic material made of urea, and an endothermic material made of trehalose.

The solubility of potassium chloride is approximately 27% to a test liquid (physiological saline) adjusted to 37°C ± 5°C. As examples of the solubility of other endothermic materials, that of urea is approximately 61% and that of trehalose is approximately 47% to the test liquid (physiological saline) adjusted to 37°C ± 5°C.

Here, it is preferable that the multiple endothermic materials of different kinds having solubility different from each other in at least not less than 10%. For example, when potassium chloride (first endothermic material) having a solubility of approximately 27% and urea (second endothermic material) having a solubility of approximately 61% are mixed with pulp to produce the absorber 30, urea dissolves mainly or completely at first excretion, and potassium chloride dissolves completely at second to third excretion.

Therefore, according to the endothermic material 50 of modification 2 of the present invention, it is possible to cause the endothermic reaction for a long time. Note that the several kinds of the endothermic materials having different solubility will not be limited to the example mentioned above.

### (Absorbent Article According to Second Embodiment of the Present Invention)

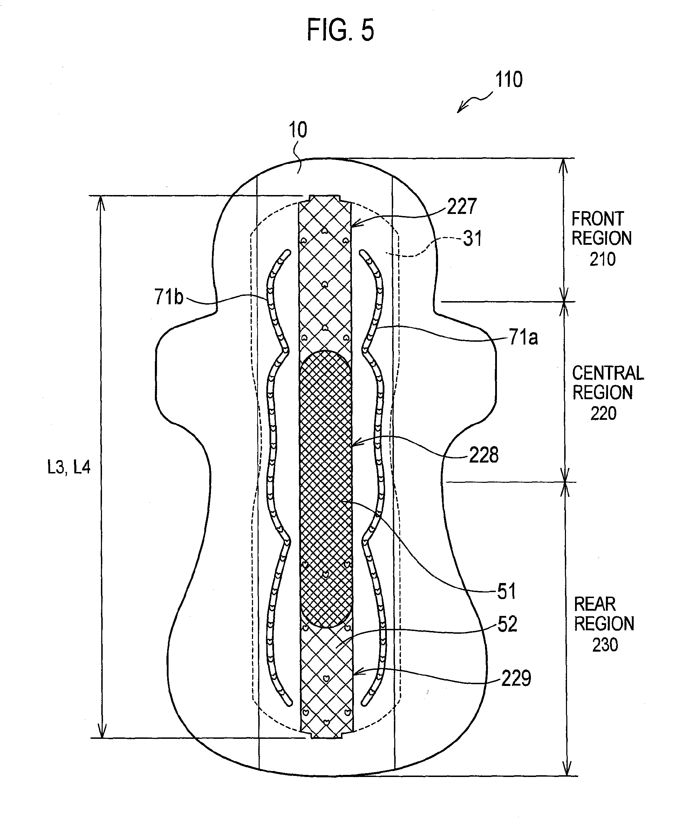

With reference to Fig. 5, a description will be given of an absorbent article according to a second embodiment of the present invention. Hereinafter, the absorbent article according to the second embodiment of the present invention will be described focusing on differences from the absorbent article according to the first embodiment of the present invention. Fig. 5 is a plan view of the absorbent article 110 according to the second embodiment of the present invention.

The absorbent article 110 according to present embodiment has a longer rear region 230 in the longitudinal direction than the absorbent article 100 according to the first embodiment. In other words, the absorbent article 110 has a longer region contacting the skin surface on the buttocks side (tailbone side) of the wearer. The absorbent article 110 is configured so as to absorb menstrual blood, etc. that flows along the body of the wearer (for example, buttocks), for example, even when the wearer is lying (for example, lying on her back).

As shown in Fig. 5, in the absorbent article 110, the embossed grooves 71a to 71b (the so-called hinge) are formed in the top sheet 10 and the absorber 31 to be in a pattern tracing a shape of the crotch part so that the absorbent article 110 is contoured to fit the crotch part.

Moreover, in the absorbent article 110 according to the present embodiment, the endothermic material is disposed in an arrangement region including at least the central region 220 of the absorbent article 110. Additionally, the mixing percentage of the endothermic material in both end portions in the longitudinal direction of the absorbent article 110 within the arrangement region is lower than the mixing percentage of the endothermic material in the central portion in the longitudinal direction of the absorbent article within the arrangement region.

Specifically, as shown in Fig. 5, in the absorbent article 110, the endothermic materials 51 to 52 are disposed across the arrangement regions 227 to 229 in the longitudinal direction of the absorber 31. In the example of Fig. 5, the arrangement regions 227 to 229 in a planar direction of the endothermic materials 51 to 52 are indicated by a mesh pattern. As shown in Fig. 5, when a length in the longitudinal direction of the absorber 30 is "L3", it is preferable that a whole length of the arrangement regions 227 to 229 in the longitudinal direction be a length "L4" which is approximately equal to a length "L3".

Here, the endothermic material 51 is the endothermic material in the central portion in the longitudinal direction of the absorbent article 110. The endothermic material 51 is disposed in the arrangement region 228, which is the central portion in the longitudinal direction. The central portion in the longitudinal direction of the absorbent article 110 denotes a region including at least the center of a whole length in the longitudinal direction of the absorbent article 110.

Moreover, the endothermic material 52 is the endothermic material in the both end portions in the longitudinal direction of the absorbent article 110 within the arrangement regions 227 to 229. The endothermic material 52 is disposed in the arrangement region 227 and the arrangement region 229. The both end portions in the longitudinal direction of the absorbent article 110 denote a belly side region and a buttocks side region relative to the region of the above-mentioned central portion when the wearer puts on the absorbent article 110.

Moreover, the mixing percentage of the endothermic material 52 in the arrangement region 227 and that of the arrangement region 229 are lower than the mixing percentage of the endothermic material 51 in the arrangement region 228. The mixing percentage denotes a mixing amount per unit area, for example, a weight of the endothermic material per unit area. Alternatively, endothermic materials having different grain diameters may be disposed in the endothermic materials 51 to 52.

In the absorbent article 11 according to the second embodiment of the present invention, the endothermic materials 51 to 52 are disposed in the arrangement regions ranging across a whole surface in the longitudinal direction of the absorber 31. Accordingly, even when the wearer excretes the body fluid such as menstrual blood to the front region 210 and the rear region 230 of the absorbent article 110, it is possible to cause the endothermic reaction more securely.

According to the absorbent article 110 of the second embodiment of the present invention, the mixing percentage of the endothermic material 52 in the both end portions in the longitudinal direction of the absorbent article 110 is lower than that of the endothermic material 51 in the central portion in the longitudinal direction. According to the absorbent article 110, the endothermic material 51 can cause sufficient endothermic reaction also in the central portion, to which a large amount of the body fluid such as menstrual blood is excreted.

Further, in the absorbent article 110 according to the second embodiment of the present invention, leakage probability of the red blood cell can decrease since the water-soluble material is dissolved into the blood that has once passed through the top sheet 10. Therefore, the blood is not easily returned to the skin surface through the top sheet 10. Thus, the wearer can feel sense of dryness.

In this way, according to the absorbent article 110 of the second embodiment of the present invention, it is possible to suppress the stuffiness that the wearer feels when the body fluid such as menstrual blood is excreted.

### (Other Embodiments)

While the sanitary napkin has been described as an example of the absorbent article in the above-mentioned embodiments, the present invention will not be limited to the sanitary napkin, and can also be applied to feminine absorbent articles such as panty liners. Further, the absorbent article can also be applied to incontinence pads and diapers.

As mentioned above, while the present invention has been described in detail using the above-mentioned embodiments, it should be obvious for those skilled in the art that the present invention should not be limited to the embodiments described herein. The present invention can be implemented as modifications and modified aspects, without departing from the spirit and scope of the present invention defined by the description of the scope of claims. Accordingly, the description herein is aimed at describing an example, and it does not have any restrictive sense to the present invention. Further, embodiments and modifications of the present invention can be combined.

### [Examples]

Next, more detailed description will be given of the present invention using examples. However, the present invention will not be limited to the following examples at all.

### <Test device>

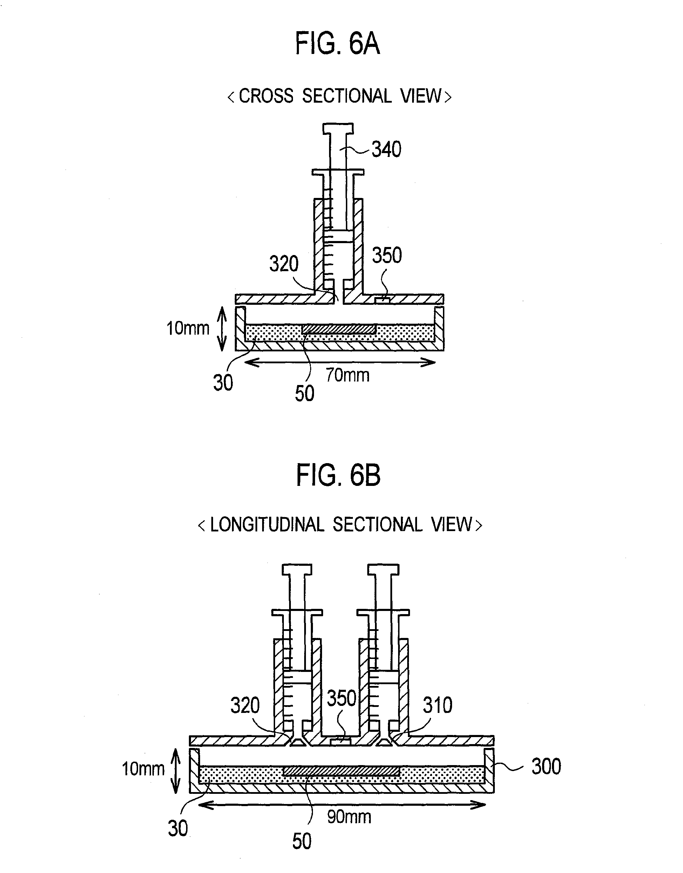

First, in order to reproduce a space between a skin and an absorbent article, and in order to reproduce a diffusion state of body fluid during wearing, a test device as shown in Fig. 6A to Fig. 6B was produced. Fig. 6A is a cross sectional view of the test device, and Fig. 6B is a longitudinal sectional view of the test device.

In the test device, an enclosed space of 90 mm long, 70 mm wide, and 10 mm deep was produced with an acrylic container 300. In order to measure changes in temperature and humidity of the enclosed space, a temperature and humidity sensor 350 was installed in the test device.

In order to diffuse a test liquid in the vertical direction, an acrylic lid was installed above the acrylic container 300, the lid provided with holes into which two syringes 340 could be inserted in a vertical direction, and holes 310 to 320 through which drops of the test liquid passed in the vertical direction to drop at two locations respectively from the syringes 340. In other words, the test liquid could be dropped from four holes aligned in the vertical direction.

In order to match with salt concentration of a body fluid, physiological saline was used as a test liquid having a composition in which distilled water is mixed with sodium chloride in a way that sodium chloride concentration might be 0.9%.

A temperature of the test liquid was adjusted to 37°C ±5°C, in order to match with a temperature of the body fluid.

### <Example 1>

Sample 1 having an absorber in which an endothermic material was disposed was produced. Specifically, first, as an upper layer material of the absorber, pulp of 150 gsm and potassium chloride of 1.5g were blended, and the mixture was formed to have a longitudinal dimension of 100 mm and a width dimension of 30 mm.

An average grain diameter of potassium chloride (endothermic material) was 425 µm, and potassium chloride in a range (range of the average grain diameter) of 325 to 525 µm was 42% of an entire volume. Grain size distribution of potassium chloride was the grain size distribution shown in Fig. 3.

Moreover, as a lower layer material of the absorber, pulp of 130 gsm and a high polymer absorbent 0.3 g were blended, and the mixture was formed to have a longitudinal dimension of 200 mm and a width dimension of 70 mm.

The upper layer material and the lower layer material thus formed were layered and covered with a tissue of 14 gsm. After covering, a thickness of the absorber was adjusted to 3.4 mm by embossing.

After embossing, an air-through nonwoven fabric of 35 gsm as a top sheet was placed on the absorber. In order to integrate the top sheet and the absorber, both sides thereof were joined by embossing.

After joining the top sheet and the absorber by embossing, in order to dispose the top sheet and the absorber on the test device, the top sheet and the absorber were cut into a longitudinal dimension of 90 mm and a width of 70 mm. The obtained product was formed as Sample 1.

As Sample 2, an absorber in which no endothermic material was disposed was produced. Sample 2 was produced in the same method as that of Sample 1 except that potassium chloride (endothermic material) was not disposed in the upper layer material of the absorber.

### (Evaluation Method 1)

An evaluation test was conducted in the following manner using Sample 1 thus produced. The evaluation test was conducted under an environment of 35°C ± 5°C and 75 RH% ±5 RH%.

First, the acrylic lid of the test device was opened, and Sample 1 was installed in the acrylic container 300. Next, the acrylic lid was closed and Sample 1 was left in the container for 15 minutes for stabilization. At the time when the acrylic lid was closed, measurements of the temperature and humidity of the enclosed space were begun.

After 15 minutes from the start of the measurements (after stabilization), 2 ml of the test liquid adjusted to 37°C ± 5°C was dropped. After 30 minutes from that, 2 ml of the test liquid at the same temperature was dropped. This process was repeated 5 times and a total of 10 ml of the test liquid was dropped. From the start of the measurements, the temperature and humidity for 2 hours and 45 minutes in total were measured.

Moreover, the above-mentioned evaluation test was similarly conducted on Sample 2 as well.

### (Evaluation Result 1)

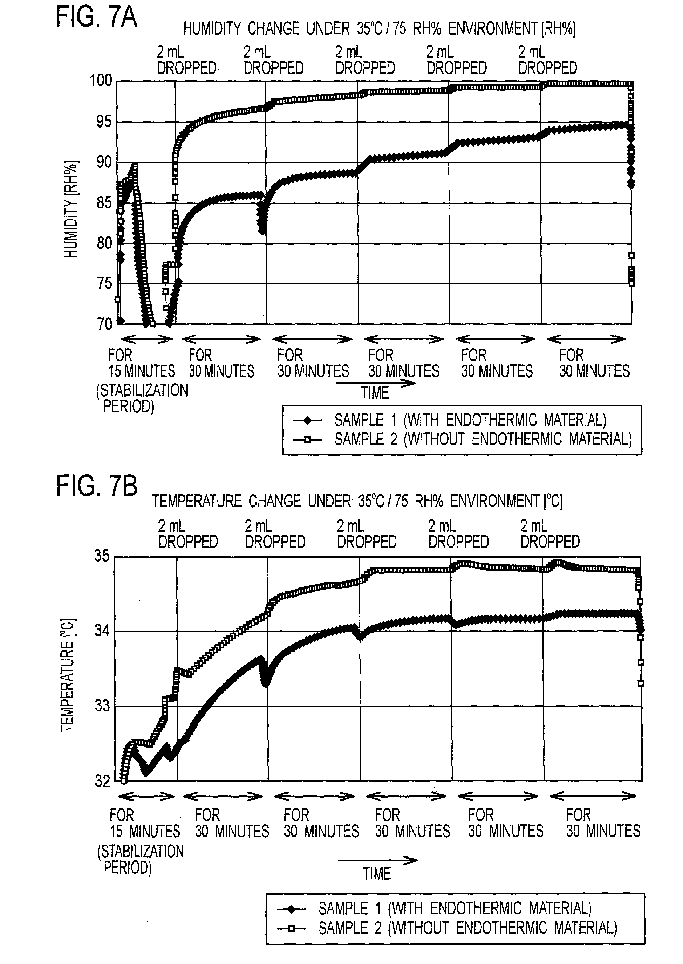

Figs. 7A to 7B show measurement results of the temperature and humidity obtained by the above-mentioned evaluation test. Fig. 7A shows the measurement results of humidity, and Fig. 7B shows the measurement results of temperature.

As shown in Figs. 7A to 7B, in Sample 2, the temperature and humidity in the enclosed space of the test device sharply rose immediately after dropping 2 ml of the test liquid. In contrast, the temperature and humidity in Sample 1 rose less than those in Sample 2. Moreover, even when 2 ml of the test liquid was repeatedly (5 times) dropped, the temperature and humidity in Sample 1 rose less than those in Sample 2.

### (Evaluation Method 2)

The following evaluation test was conducted using obtained Sample 1. The evaluation test was conducted under an environment of 35°C ± 5°C and 75 RH% ± 5 RH%.

First, the acrylic lid of the test device was opened, and Sample 1 was installed in the acrylic container 300. Next, the acrylic lid was closed and Sample 1 was left in the container for 15 minutes for stabilization. At the time when the acrylic lid was closed, measurements of the temperature and humidity of the enclosed space were begun.

After 15 minutes from the start of the measurements (after stabilization), 6 ml of the test liquid adjusted to 37°C ± 5°C was dropped. Subsequently, the temperature and humidity for 60 minutes were measured.
Moreover, the above-mentioned evaluation test was similarly conducted on Sample 2 as well.

### (Evaluation Result 2)

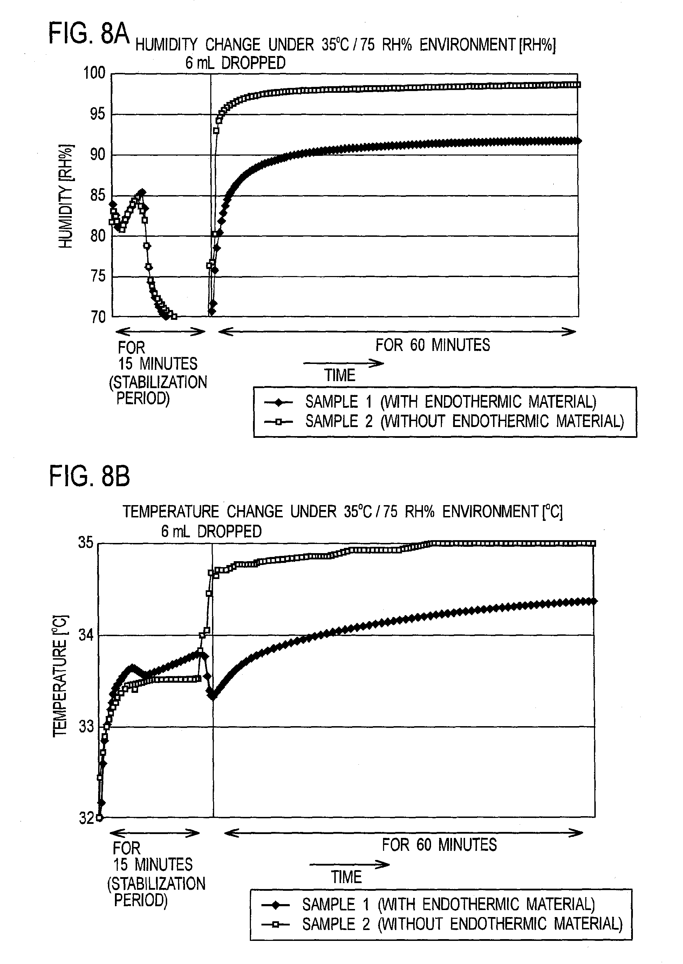

Figs. 8A to 8B show measurement results of the temperature and humidity obtained by the above-mentioned evaluation test. Fig. 8A shows the measurement results of humidity, and Fig. 8B shows the measurement results of temperature.

As shown in Figs. 8A to 8B, in Sample 2, the temperature and humidity sharply rose immediately after dropping. In contrast, the temperature and humidity in Sample 1 rose less than those in Sample 2.

### (Consideration Concerning Example 1)

The evaluation tests and evaluation results mentioned above showed that Sample 1 can suppress sharp increases in temperature and humidity in the space between the skin surface and the absorbent article, even when excretion of the body fluid is repeated, or even when a large amount of the body fluid is excreted. Since the temperature of the enclosed space in Sample 1 does not reduce too much from the temperature of Sample 2, it also turns out that reduction in temperature is in a level which gives no uncomfortable feelings such as chilliness to the wearer.

As mentioned above, the absorbent article according to the present invention can suppress increases in temperature and humidity in the space between the skin surface and the absorbent article when the body fluid such as menstrual blood or urine is excreted. Accordingly, the absorbent article can suppress the stuffiness that the wearer feels.

### <Example 2>

Using potassium chloride as an example, an evaluation test was conducted to find speeds at which potassium chloride having different grain diameters completely dissolves by hydration.

First, multiple endothermic materials having different grain diameters were produced as samples. Specifically, an endothermic material of a grain diameter within the range of "600 to 710 µm" (the range of a larger grain diameter) was produced as Sample A. An endothermic material of a grain diameter within the range of "355 to 500 µm" (the range of an average grain diameter) was produced as Sample B. Further, an endothermic material of a grain diameter within the range of "150 to 250 µm" (the range of a smaller grain diameter) was produced as Sample C.

Moreover, a test liquid was produced. Specifically, in order to match a composition of the test liquid with salt concentration of a body fluid, physiological saline was produced as the test liquid by mixing distilled water with sodium chloride in a way that sodium chloride concentration might be 0.9%.

### (Evaluation method)

First, a temperature of the test liquid was adjusted to 37°C ± 5°C, in order to match the temperature of the test liquid with a temperature of the body fluid.

Next, 1.0 g of each Sample A to C was extracted, and placed into a glass beaker (for 50 ml with a diameter of 43 mm).
Moreover, 3.0 ml of the test liquid was dropped into the glass beaker.
A measurement was made for each sample on the time from the test liquid was dropped until completely dissolved.

### (Evaluation results)

The following measurement results were obtained from the above-mentioned evaluation test.

Sample A: range of a larger grain diameter (range of 600 to 710 µm): 62 minutes and 10 seconds

Sample B: range of the average grain diameter (range of 355 to 500 µm) 52 minutes and 43 seconds

Sample C: range of a smaller grain diameter (range of 150 to 250 µm): 25 minutes and 37 seconds

### (Consideration concerning Example 2)

According to the evaluation test and evaluation results mentioned above, it turned out that an endothermic material having a larger grain diameter tends to be completely dissolved more slowly, although it may depend on an amount of the test liquid dropped or the temperature and humidity of the test environment. In other words, it turned out that mixing endothermic materials having different grain diameters enables the endothermic reaction to appear for a long time.

Note that whole contents of Japan Patent Application No. 2008-140053 (filed on May 28, 2008) are incorporated herein by reference.

### Industrial Applicability

As described above, according to the present invention, it is applicable for an absorbent article and a sanitary napkin each of which can suppress the stuffiness that the wearer feels when a body fluid such as menstrual blood or urine is excreted.

## Claims

1. An absorbent article comprising:
a liquid permeable top sheet;
a liquid impermeable back sheet; and
an absorber interposed between the top sheet and the back sheet,
wherein
in the absorbent article, a front region, a central region, and a rear region are provided continuously in a longitudinal direction of the absorber, and
an endothermic material is disposed inside the absorber and in an arrangement region including at least the central region of the absorbent article.

2. The absorbent article according to claim 1, wherein a mixing percentage of the endothermic material in both end portions in a longitudinal direction of the absorbent article within the arrangement region is lower than a mixing percentage of the endothermic material in the central portion in the longitudinal direction of the absorbent article within the arrangement region.

3. The absorbent article according to claim 1, wherein the endothermic material is disposed in a plurality having different grain diameters.

4. The absorbent article according to claim 3, wherein the endothermic material having a grain diameter within a range of ± 100 µm of an overall average grain diameter is not more than 60% of the endothermic materials disposed inside the absorber.

5. The absorbent article according to claim 3, wherein a grain diameter of the endothermic material disposed inside the absorber on a side facing the top sheet is smaller than a grain diameter of the endothermic material disposed inside the absorber on a side facing the back sheet.

6. A sanitary napkin comprising:
a liquid permeable top sheet;
a liquid impermeable back sheet; and
an absorber interposed between the top sheet and the back sheet,
wherein
in the sanitary napkin, a front region, a central region, and a rear region are provided continuously in a longitudinal direction of the absorber, and
an endothermic material is disposed inside the absorber in an arrangement region including at least the central region of the sanitary napkin.

7. The sanitary napkin according to claim 6, wherein
the sanitary napkin further includes a wing extending in a width direction, and
a region in a longitudinal direction of the central region is a region in a longitudinal direction of the wing.

8. The sanitary napkin according to claim 6, wherein
in the sanitary napkin, an embossed groove is formed in both side portions in a width direction of the absorber,
the embossed groove is formed along a longitudinal direction of the sanitary napkin, and
the endothermic material is interposed between the embossed grooves.
